**Europäisches Patentamt**

(19) **European Patent Office**

**Office européen des brevets**

(11) Veröffentlichungsnummer: **0 006 156**
**B1**

# EUROPÄISCHE PATENTSCHRIFT

(45) Veröffentlichungstag der Patentschrift:
01.07.81

(21) Anmeldenummer: 79101658.7

(22) Anmeldetag: 30.05.79

(51) Int. Cl.³: **C 07 D 307/32,** C 07 D 307/60,
C 07 B 3/00

(54) Verfahren zur Herstellung von Dihydro-4,4-dimethyl-2,3-furandion.

(30) Priorität: 30.05.78 CH 5883/78

(43) Veröffentlichungstag der Anmeldung:
09.01.80 Patentblatt 80/1

(45) Bekanntmachung des Hinweises auf die Patenterteilung:
01.07.81 Patentblatt 81/26

(84) Benannte Vertragsstaaten:
AT BE CH DE FR GB IT NL

(56) Entgegenhaltungen:
AT-B-337 663
DD-A-32 628
DD-A-37 505
DD-A-41 651
DE-A-1 568 755
DE-B-1 033 652
CH-A-471 111
BEILSTEINS HANDBUCH DER ORGANISCHEN
CHEMIE, 4. Auflage, Band 17, Seite 5848.
BEILSTEINS HANDBUCH DER ORGANISCHEN
CHEMIE, 4. Auflage, Band 16, Seite 23.

(73) Patentinhaber: **F. HOFFMANN-LA ROCHE & CO.
Aktiengesellschaft, CH-4002 Basel (CH)**

(72) Erfinder: **Schmid, Max, Dr., Stapferstrasse 21,
CH-5200 Brugg. (CH)**

(74) Vertreter: **Lederer, Franz, Dr. et al, Patentanwälte Dr.
Franz Lederer Reiner F. Meyer Lucile-Grahn-Strasse 22,
D-8000 München 80 (DE)**

## Verfahren zur Herstellung von Dihydro-4,4-dimethyl-2,3-furandion

Die vorliegende Erfindung betrifft ein Verfahren zur Herstellung eines Diketons, und zwar von Dihydro-4,4-dimethyl-2,3-furandion.

Diese Verbindung, welche auch als Ketopantolacton bezeichnet wird, stellt ein wichtiges Ausgangsmaterial für die Herstellung von optisch aktivem Dihydro-3-hydroxy-4,4-dimethyl-2(3H)-furanon (Pantolacton) dar.

Das Dihydro-4,4-dimethyl-2,3-furandion kann durch Oxydation von Dihydro-3-hydroxy-4,4-dimethyl-2(3H)-furanon erhalten werden, wobei bisher als Oxydationsmittel Bleitetraacetat, N-Bromsuccinimid und CrO₃ (Jones-Reagens) verwendet wurden. Diesen Oxydationsverfahren gemeinsam ist die relativ geringe Ausbeute sowie die Verwendung teurer Oxydationsmittel.

Es hat sich nun gezeigt, daß man das gewünschte Dihydro-4,4-dimethyl-2,3-furandion in wesentlich höheren Ausbeuten als bisher und unter Verwendung billigerer Oxydationsmittel erhält, wenn man erfindungsgemäß die Oxydation mit einem festen Alkali- oder Erdalkalimetallhypochlorit in organischer Phase durchführt.

Es handelt sich bei dieser Oxydation somit um eine heterogene Oxydation, bei welcher das Oxydationsmittel in der flüssigen Phase praktisch unlöslich ist.

Als Oxydationsmittel kann zweckmäßig Calciumhypochlorit, Bariumhypochlorit oder Lithiumhypochlorit verwendet werden, wobei die Verwendung von Calciumhypochlorit bevorzugt ist.

Der Begriff Calciumhypochlorit umfaßt auch Chlorkalk, welcher zu einem wesentlichen Teil aus Calciumhypochlorit besteht.

Die Natur der vorliegenden Oxydation (heterogene Oxydation) bedingt, daß als Lösungsmittel ein solches verwendet wird, worin das Ausgangsmaterial und das Endprodukt löslich sind, worin jedoch das Oxydationsmittel praktisch unlöslich ist. Als derartige Lösungsmittel kommen in Frage Kohlenwasserstoffe bzw. chlorierte Kohlenwasserstoffe, welche selbst oxydationsunempfindlich sind, wie beispielsweise Benzol, Methylenchlorid, Chloroform, Hexan, sowie Äther, Acetonitril und dergleichen. Bevorzugte Lösungsmittel sind Benzol, Methylenchlorid und Acetonitril.

Das als Oxydationsmittel verwendete Hypochlorit wird vor seinem Einsatz zweckmäßig getrocknet, wobei es nicht notwendig ist, beispielsweise im Falle von Calciumhypochlorit (3 Mol Kristallwasser), das Kristallwasser zu entfernen.

Das als Oxydationsmittel verwendete Hypochlorit wird zweckmäßig in etwa äquimolaren Mengen oder in geringem Überschuß verwendet.

Die verwendete Menge an Lösungsmittel sollte ausreichen, um das gesamte Ausgangsmaterial zur Lösung zu bringen und ein noch rührbares Reaktionsgemisch zu erhalten.

Die erfindungsgemäße Oxydation wird zweckmäßig bei Temperaturen von etwa 0 bis etwa 40°C, vorzugsweise bei Raumtemperatur, durchgeführt.

Die Reaktionszeit liegt im allgemeinen bei wenigen Stunden, beispielsweise zwischen etwa 4 Stunden und etwa 15 Stunden.

Durch Filtrieren, des erhaltenen Reaktionsgemisches erhält man eine Lösung, welche nach Eindampfen das gewünschte Dihydro-4,4-dimethyl-2,3-furandion in Ausbeuten von etwa 70—90% ergibt.

### Beispiel 1

In einem mit einem mechanischen Rührer, einem Rückflußkühler, einem Tropftrichter und einem Thermometer ausgestatteten Kolben von 1 l Inhalt werden 107,5 g eines im Handel befindlichen, vorher getrockneten Calciumhypochlorit-Präparates, entsprechend 78 g Calciumhypochlorit, und 500 ml von über Molekularsieb getrocknetem Acetonitril vorgelegt.

Dieser Mischung wird unter kräftigem Rühren eine Lösung von 63,4 g racemischem Dihydro-3-hydroxy-4,4-dimethyl-2(3H)-furanon(DL-Pantolacton) in 250 ml trockenem Acetonitril innerhalb von 30 Minuten zugetropft.

Anschließend wird die Reaktionsmischung noch während 4 Stunden bei Raumtemperatur gerührt, durch eine Glasfritte filtriert und der Rückstand mit 150 ml Acetonitril gewaschen. Nach Entfernung des Lösungsmittels am Rotationsverdampfer und Trocknen unter vermindertem Druck erhält man 49 g des gewünschten Dihydro-4,4-dimethyl-2,3-furandion mit einem Schmelzpunkt von 61—64°C. Nach Umkristallisation aus Äther erhält man 45 g der reinen Substanz mit einem Schmelzpunkt von 66—67°C.

Das hierbei verwendete Calciumhypochlorit-Präparat wurde vor seiner Verwendung durch Trocknen eines im Handel befindlichen Produktes im Trockenschrank bei 60—70°C und Hochvakuum während 24 Stunden erhalten. Dieses im Handel befindliche Präparat enthielt 65% Calciumhypochlorit, 15% Natriumchlorid, 10% Wasser und etwa 10% Calciumcarbonat und andere Verunreinigungen.

Anstelle dieses Produktes kann man auch das im Handel befindliche Calciumhypochlorit mit 3 Mol Kristallwasser oder ein Präparat mit einem geringeren Kristallwassergehalt verwenden.

Als Oxydationsmittel kann auch getrockneter und zerkleinerter Chlorkalk mit einem Calciumhypochloritgehalt von etwa 30% verwendet werden.

### Beispiel 2

In einem wie im Beispiel 1 beschriebenen Kolben wird zu einer Mischung aus 136,9 g

Calciumhypochlorit [Ca(OCl)$_2$ · 2 H$_2$O (65%ig)] und 500 ml Methylenchlorid eine Lösung von 65 g DI-Pantolacton in 250 ml Methylenchlorid unter kräftigem Rühren innerhalb von 40 Minuten zugetropft, wobei die Innentemperatur auf etwa 35° C ansteigt. Die Reaktionsmischung wird noch über Nacht bei Raumtemperatur gerührt und anschließend durch eine Glasfritte filtriert. Der Rückstand wird mit 200 ml frischem Methylenchlorid gewaschen. Nach Entfernung des Lösungsmittels im Rotationsverdampfer und Trocknung beträgt die Ausbeute an Ketopantolacton 48 g (75%). Dieses kann durch Umkristallisation aus Äther gemäß Beispiel 1 gereinigt werden.

## Beispiel 3

In einem mit einem mechanischen Rührer, einem Rückflußkühler, einem Tropftrichter und einem Thermometer ausgestatteten Kolben von 500 ml Inhalt werden 80 g getrocknetes Lithiumhypochlorit (~35% aktives Chlor, 30% LiOCl) und 300 ml trockenes Benzol vorgelegt. Dieser Mischung wird unter kräftigem Rühren eine Lösung von 26 g racemischem Dihydro-3-hydroxy-4,4-dimethyl-2(3H)-furanon (DL-Pantolacton) in 100 ml trockenem Benzol innerhalb von 30 Minuten zugetropft. Das Gemisch wird über Nacht weiter gerührt und dann in zu Beispiel 1 analoger Weise aufgearbeitet und man erhält das gewünschte Dihydro-4,4-dimethyl-2,3-furandion.

## Patentansprüche

1. Verfahren zur Herstellung von Dihydro-4,4-dimethyl-2,3-furandion durch Oxydation von Dihydro-3-hydroxy-4,4-dimethyl-2(3H)-furanon, dadurch gekennzeichnet, daß man die Oxydation mit einem festen Alkali- oder Erdalkalimetallhypochlorit in organischer Phase durchführt.

2. Verfahren nach Anspruch 1, dadurch gekennzeichnet, daß man als Oxydationsmittel Calciumhypochlorit, Bariumhypochlorit oder Lithiumhypochlorit verwendet.

3. Verfahren nach Anspruch 1 oder 2, dadurch gekennzeichnet, daß man als Lösungsmittel Benzol, Methylenchlorid, Chloroform oder Acetonitril verwendet.

4. Verfahren nach Anspruch 1, 2 oder 3, dadurch gekennzeichnet, daß man die Oxydation bei einer Temperatur von etwa 0 bis etwa 40° C, vorzugsweise bei Raumtemperatur, durchführt.

## Claims

1. A process for the manufacture of dihydro-4,4-dimethyl-2,3-furandione by oxidising dihydro-3-hydroxy-4,4-dimethyl-2(3H)-furanone, characterised in that the oxidation is carried out with a solid alkali metal hypochlorite or alkaline earth metal hypochlorite in an organic phase.

2. A process according to claim 1, characterised in that calcium hypochlorite, barium hypochlorite or lithium hypochlorite is used as the oxidising agent.

3. A process according to claim 1 or 2, characterised in that benzene, methylene chloride, chloroform or acetonitrile is used as the solvent.

4. A process according to claim 1, 2 or 3, characterised in that the oxidation carried out at a temperature of about 0 to about 40° C, preferably at room temperature.

## Revendications

1. Procédé de préparation de la dihydro-4,4-diméthyl-2,3-furanne-dione par oxydation de la dihydro-3-hydroxy-4,4-diméthyl-2(3H)-furan-none, caractérisé en ce que l'on effectue l'oxydation à l'aide d'un hypochlorite de métal alcalin ou alcalino-terreux solide en phase organique.

2. Procédé selon la revendication 1, caractérisé en ce que l'on utilise en tant qu'agent oxydant l'hypochlorite de calcium, l'hypochlorite de baryum ou l'hypochlorite de lithium.

3. Procédé selon la revendication 1 ou 2, caractérisé en ce que l'on utilise comme solvant le benzène, le chlorure de méthylène, le chloroforme ou l'acétonitrile.

4. Procédé selon la revendication 1, 2 ou 3, caractérisé en ce que l'on effectue l'oxydation à une température de 0 à 40° C environ, de préférence à température ambiante.